# EUROPÄISCHE PATENTSCHRIFT

(11) **EP 2 818 094 B1**
(45) Veröffentlichungstag und Bekanntmachung des Hinweises auf die Patenterteilung: **29.07.2020**
(21) Anmeldenummer: 14172499.7
(22) Anmeldetag: 16.06.2014
(51) Int. Cl.: A61B 1/00, A61B 1/05

(54) **BEOBACHTUNGSINSTRUMENT MIT EINEM SYMMETRISCHEN BILDFELD UNTER VERWENDUNG ASYMMETRISCHER BILDSENSOREN**
OBSERVATION INSTRUMENT WITH A SYMMETRICAL FIELD OF VIEW USING ASYMMETRIC IMAGE SENSORS
INSTRUMENT D'OBSERVATION ÉQUIPÉ D'UN CHAMP D'IMAGE SYMÉTRIQUE EN UTILISANT DES CAPTEURS D'IMAGE ASYMÉTRIQUES

(30) Priorität: 17.06.2013 DE 102013106278
(43) Veröffentlichungstag der Anmeldung: 31.12.2014
(73) Patentinhaber: Karl Storz SE & Co. KG, 78532 Tuttlingen (DE)
(72) Erfinder: BAUMANN, Harald, 78532 Tuttlingen (DE); SCHWARZ, Peter, 78532 Tuttlingen-Nendingen (DE)
(74) Vertreter: Witte, Weller & Partner Patentanwälte mbB

(56) Entgegenhaltungen:
- EP-A1- 1 685 790
- EP-A1- 2 719 321
- EP-A2- 2 392 248
- DE-A1- 3 736 688
- GB-A- 778 276
- JP-A- H09 122 071
- US-A- 3 901 220
- US-A- 4 890 159
- US-A- 4 918 521
- US-A- 5 912 764
- US-A1- 2008 091 064

## Beschreibung

Die Erfindung betrifft ein Beobachtungsinstrument, mit einem eine Mittelebene aufweisenden Hohlschaft, in dessen distalem Endbereich ein opto-elektronisches Bildaufnehmersystem angeordnet ist, das bildeintrittseitig ein distales Umlenkprisma aufweist, dessen Umlenkflächen ein Bild in ein optisches Linsensystem leiten, das das Bild auf einen proximal vom Linsensystem angeordneten Bildsensor auf dessen Bildebene abbildet, wobei der Bildsensor einen aktiven Bereich aufweist, der asymmetrisch zur Außenkontur des Bildsensors liegt, wobei der Bildsensor gekippt gegenüber einer Längsachse des Schaftes angeordnet ist, wobei zwischen dem proximalen Ende des Linsensystems und dem Bildsensor ein proximales Umlenkprisma angeordnet ist, das das vom optischen Linsensystem austretende Bild auf den gekippten Bildsensor umlenkt, und wobei der Hauptstrahl zentral mittig längs einer Längsachse des Linsensystems geführt wird.

Ein derartiges Beobachtungsinstrument in Form eines medizinischen Endoskopes ist bspw. aus der US 4 720 178 A bekannt. Aus der US 4 809 680 A ist bekannt, dass Bildsensoren einen aktiven Bereich aufweisen, der asymmetrisch zur Außenkontur des Bildsensors liegt.
Aus der US 4 890 159 A ist ein Endoskopiesystem bekannt, in dem ein Bild vereinheitlicht wird; mit einer Mehrzahl von Endoskopen, welche einen Einführschaft, ein optisches Abbildungssystem an der Spitze des Einführschaftes und eine Bilderfassungseinheit zur Zeugung eines Bildes aufweisen, wobei sich zumindest eines der Mehrzahl von Endoskopen von den anderen hinsichtlich der Auslegung unterscheidet, ob ein seitenrichtiges oder seitenverkehrtes Bild erzeugt wird; mit einer Signalverarbeitungseinrichtung, die gemeinsam mit der Mehrzahl von Endoskopen verwendet wird, wobei die Signalverarbeitungseinrichtung ein Videosignal erzeugt und unabhängig davon, ob das Bild seitenrichtig oder seitenverkehrt ist, den gleichen Verarbeitungsprozess nutzt; und mit einer Vereinheitlichungseinheit, die zur Vereinheitlichung des Bildes an einem Signalausgang vorgesehen und die als Gegenstück zu jedem der Endoskope ausgebildet ist, um das Bild am Signalausgang zu vereinheitlichen.
Aus der EP 2 392 248 A2 ist ein Schrägblick-Endoskop bekannt, mit einer Objektivbaugruppe, die ein distales Prima mit Umlenkflächen umfasst, wobei die Objektivbaugruppe einfallendes Licht einem Bildaufnehmer mit einem Sensor zuführt, der in einem Schaft des Endoskopes steht.
Weitere Gestaltungen von Beobachtungsinstrumenten mit distalen Prismen sind aus der US 5 912 764 A, US 3 901 220 A, GB 778 276 A und der EP 1 685 790 A1 bekannt.

Üblicherweise zeigen Bildsensoren eine viereckige, meist eine rechteckige Außenkontur auf. Aus konstruktiven Gründen, insbesondere zur Aufnahme der zahlreichen elektronischen Elemente, liegt der aktive Bereich asymmetrisch, d.h. meist seitlich, nach außen versetzt. Der aktive Bereich weist ebenfalls meist eine rechteckförmige Kontur auf.

Je nach den Größenverhältnissen und den konstruktiven Gegebenheiten im Schaft, kann ein solcher Bildsensor stehend oder gekippt, oder liegend eingebaut werden.

Ist er stehend eingebaut, verläuft seine Bildebene senkrecht zur Längsachse des Schaftes. Bei gekippten Anordnungen ist diese Bildebene gegenüber dieser Längsachse etwas gekippt. Beim liegenden Einbau verläuft die Bildebene des Bildsensors in Richtung der Längsachse und meist seitlich versetzt parallel dazu.

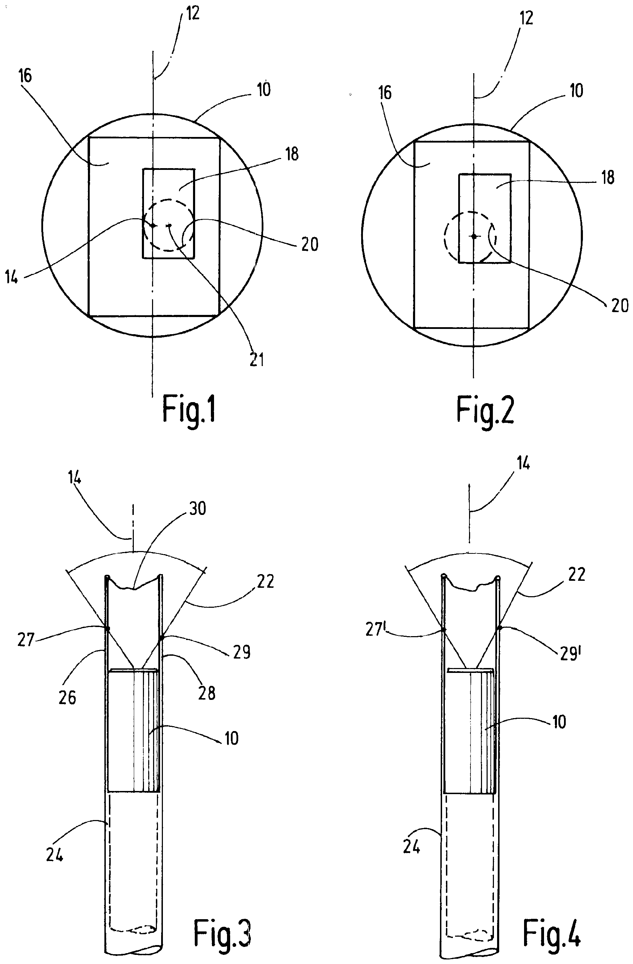

Für eine optimale Ausnutzung der Bildinformation ist es notwendig, dass das Bild so auf den aktiven Bereich des Bildsensors abgebildet wird, dass das Bild innerhalb dieses aktiven Bereiches liegt. Dies ist in Fig. 1 dargestellt. Dort ist ersichtlich, dass ein Bildsensor 16 mit einem asymmetrischen aktiven Bereich 18 stehend in einem Schaft 10 eingebaut ist. Soll die Bildfläche 20 optimal vom aktiven Bereich 18 erfasst werden, wobei diese Bildfläche meist kreisförmig ist, liegt der Hauptstrahl 21 seitlich versetzt zur Mittellängsachse 14 des Schaftes 10 somit seitlich auch gegenüber dessen Mittelebene 12 versetzt. Damit wäre auch ein asymmetrischer Sichtkegel des Beobachtungsinstrumentes vorhanden.

Ein asymmetrischer Sichtkegel 22 wie er bspw. in Fig. 3 dargestellt ist, hat aber gewisse Nachteile. In Fig. 3 ist ein Schaft 10 eines Endoskopes dargestellt, über den ein Überschaft 24 geschoben ist. Vom distalen Ende des Überschaftes 24 stehen zwei stangenförmige Elemente 26 und 28 vor, die am distalen Ende über eine Resektoskopschlinge 30 verbunden sind. Bei einem asymmetrischen Sichtkegel 22 sieht die Beobachtungsperson aufgrund der Asymmetrie des Sichtkegels 22 zwei axial zueinander versetzte Stellen 27 und 29 der Stangen 26 und 28. Das ist unerwünscht, denn die Beobachtungsperson sieht dann die Stellen bzw., wenn der Schaft 10 weiter nach distal verschoben ist, die Resektoskopschlinge 30 nicht gleichzeitig oder gleichmäßig bzw. auf einer Seite unscharf. Daher sind symmetrische Sichtkegel 22 erwünscht, wie das in Fig. 4 dargestellt ist. Dabei liegen dann die Sichtstellen 27' und 29' auf derselben axialen Höhe.

Das kann aber zur Folge haben, wie das in Fig. 2 dargestellt ist, dass bei einer symmetrisch ausgebildeten Abbildungsoptik die Bildfläche 20 nicht mehr vollumfänglich im aktiven Bereich 18 des asymmetrischen Bildsensors 16 liegt.
US 4,918,521 offenbart ein Endoskop, in dessen einführbaren Abschnitt eine festkörperbasierte Bildaufnahmevorrichtung angeordnet ist, wobei die Bildaufnahmevorrichtung ein bilderzeugendes optisches System aufweist.
DE 37 36 688 A1 offenbart eine Bilderzeugungsvorrichtung mit einem Kamerakopf und einer Kamerasteuereinheit. Ein optisches Objektivsystem ist in einem vorderen Endbereich des Kamerakopfes angeordnet.
JP H09122071 A offenbart ein Endoskop mit einem Hohlschaft, in dessen distalen Endbereich ein opto-elektronisches Bildaufnehmersystem angeordnet ist, das ein Bild in ein optisches Linsensystem leitet, wobei das optische Linsensystem das Bild auf einen CCD-Sensor abbildet.

Es ist daher Aufgabe der vorliegenden Erfindung hier Abhilfe zu schaffen und ein Beobachtungsinstrument zu schaffen, bei dem bei asymmetrischen Bildsensoren ein möglichst symmetrischer Sichtkegel möglich ist und das Bild möglichst vollständig im aktiven Bereich des Bildsensors liegt.

Die Aufgabe wird durch ein Endoskop gemäß dem Gegenstand des Anspruchs 1 gelöst.

Die Verdrehung dieser beiden Ebenen zueinander um den Hauptstrahl führt dazu, dass der aktive Bereich des asymmetrischen Bildsensors durch diese Drehung in eine Position bringbar ist, in der ein zur Mittelebene des Hohlschafts symmetrischer Sichtkegel ein Bild in den aktiven Bereich abbildet. Durch das Wechselspiel der Ebenen, die einerseits durch die Konstruktion des distalen Umlenkprismas und andererseits durch die Asymmetrie des Bildsensors bestimmt sind, und deren Verdrehung erlaubt es nunmehr den zur Mittelebene symmetrischen Sichtkegel zu erlauben, so dass die eingangs genannten Probleme, wie sie in Zusammenhang mit der Resektoskopschlinge bestehen, nicht mehr auftreten können. Andererseits erfolgt durch eine entsprechende Relativverdrehung eine solche Stellung zwischen den Ebenen, dass dieser symmetrische Sichtkegel auf den bezüglich der Außenkontur des Bildsensors asymmetrischen aktiven Bereich abbildet.

In einer weiteren Ausgestaltung ist der Bildsensor stehend im Hohlschaft angeordnet.

Diese Maßnahme hat den Vorteil, dass bei der Montage der stehend angeordnete Bildsensor soweit um die Mittellängsachse des Schaftes verdreht werden kann, bis das von dem distalen Umlenkprisma kommende Bild voll im aktiven Bereich des Bildsensors liegt. Dadurch kann ein hochaufgelöstes Bild unter optimaler Ausnutzung der Auflösung des aktiven Bereiches des Bildsensors erzeugt werden, das einen symmetrischen Sichtkegel erlaubt.

Der Bildsensor ist gegenüber der Längsachse des Schaftes gekippt angeordnet und zwischen dem proximalen Ende des Linsensystems und dem Bildsensor ist ein proximales Umlenkprisma angeordnet, das das vom optischen Linsensystem austretende Bild auf den gekippten Bildsensor umlenkt.

Diese an sich bekannte Maßnahme hat den Vorteil, dass auch in relativ dünne Schäfte solche Bildsensoren eingebaut werden können.

In einer weiteren Ausgestaltung der Erfindung ist der Bildsensor liegend und parallel zur Längsachse angeordnet und das proximale Umlenkprisma bewirkt eine 90°-Umlenkung des Bildes.

Diese Maßnahme hat den Vorteil, dass auch in extrem dünne Schäfte, sich länglich erstreckende rechteckige Bildsensoren eingebaut werden können.

In einer weiteren Ausgestaltung der Erfindung sind die erste und die zweite Ebene derart zueinander verdreht, dass der Sichtkegel symmetrisch zur Schaftachse verläuft.

Diese Maßnahme hat den Vorteil, dass trotz der Verdrehung der Ebenen zueinander, ein zur Mittellängs- bzw. Schaftachse symmetrischer Sichtkegel erzeugt wird. Ein solcher Sichtkegel mit dieser hohen Symmetrie erlaubt einen Rundumblick der in allen Objektebenen ein gleich scharfes Bild ergibt.

In einer weiteren Ausgestaltung der Erfindung ist der Bildsensor mit einer Bildverarbeitungseinheit verbunden, die das durch die Verdrehung verdrehte und ggf. verschobene Bild wieder seiten- und höhenrichtig aufrichtet.

Durch die Verdrehung der beiden Ebenen zueinander wird das Bild um den entsprechenden Betrag verdreht auf den Bildsensor abgebildet. Je nachdem, wie das erste Umlenkprisma ausgestaltet ist und wie der aktive Bereich im Bildsensor liegt, kann auch das Bild entsprechend verschoben sein.

Da in der modernen Operationstechnik das Bild meist auf einem Monitor dargestellt wird, ist es für den Operateur zielführend, wenn er das Bild so sieht wie es im Objektfeld vorliegt. Das erleichtert erheblich die Handhabung des Beobachtungsinstrumentes durch den Operateur.

In einer weiteren Ausgestaltung der Erfindung ist das opto-elektronische Bildaufnehmersystem derart aufgebaut, dass eine von der 0°-Blickrichtung abweichende Blickrichtung erfolgt.

Diese Maßnahme hat den Vorteil, dass insbesondere bei von der 0°-Richtung abweichenden Blickrichtungen ein hochau fgelöstes Bild erzeugt werden kann. Das distale Umlenkprisma ist dazu vorgesehen, das von der 0°-Blickrichtung abweichende eintretende Bild so umzulenken, dass es möglichst zentral auf das meist stabförmige optische Linsensystem abgebildet wird. Somit können auch bei relativ großen Abweichungen aus der 0°-Richtung, also bspw. 30°, 4 5°oder 60°und gleichzeitig Verwendung von asymmetrischen Bildsensoren, sehr scharfe und hochauflösende Bilder erzeugt werden.

Es versteht sich, dass die vorstehend genannten und die nachstehend noch zu erläuternden Merkmale nicht nur in den angegebenen Kombination, sondern auch in anderen Kombinationen einsetzbar sind, ohne den Rahmen der vorliegenden Erfindung zu verlassen.

Die Erfindung wird anhand einiger ausgewählter Ausführungsbeispiele in Zusammenhang mit den beiliegenden Zeichnungen näher beschrieben und erläutert. Es zeigen:
- Fig. 1: stark schematisiert einen stehenden Einbau eines asymmetrischen Bildsensors in einen Schaft, wobei die Bildfläche asymmetrisch zur Mittelebene und zur Längsachse ist;
- Fig. 2: eine Darstellung, bei der bei derselben Bauweise die Bildfläche zwar symmetrisch zur Mittelebene und Längsachse des Schaftes verläuft, aber nicht mehr voll den aktiven Bereich des Bildsensors trifft;
- Fig. 3: stark schematisch einen Schaft eines Endoskopes mit asymmetrischem Sichtkegel über den ein Überschaft mit einer Resektoskopschlinge geschoben ist;
- Fig. 4: eine der Fig. 3 entsprechenden Ansicht mit symmetrischem Sichtkegel;
- Fig. 5: einen Querschnitt durch einen Schaft eines ersten Ausführungsbeispiels eines erfindungsgemäßen Beobachtungsinstruments mit stehend eingebautem Bildsensor;
- Fig. 6: einen Längsschnitt eines zweiten Ausführungsbeispiels eines erfindungsgemäßen Beobachtungsinstruments in dessen distalem Endbereich;
- Fig. 7: die Lage der Bilder einmal im Objektfeld, einmal in der Bildebene des Bildsensors entsprechend verdreht und auf dem Monitor wieder aufgerichtet;
- Fig. 8: ein Schnitt längs der Linie VIII-VIII in Fig. 6;
- Fig. 9: ein Schnitt längs der Linie IX-IX in Fig. 6;
- Fig. 10: einen Schnitt längs der Linie X-X in Fig. 6;
- Fig. 11: eine perspektivische Darstellung des zusammengesetzten optoelektronischen Bildaufnehmersystemes, und
- Fig. 12: eine der Fig. 11 entsprechende Darstellung, wobei die erste und die zweite Ebene dargestellt sind, sowie deren Maß der Verdrehung zueinander.

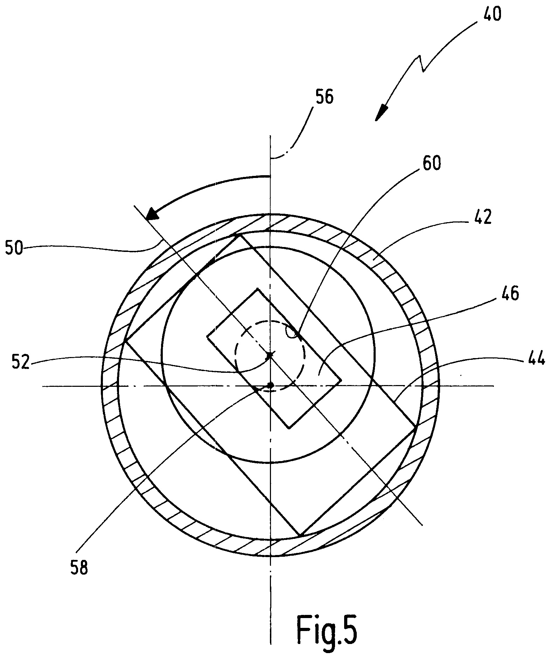

Anhand des in Fig. 5 dargestellten ersten Ausführungsbeispieles eines erfindungsgemäßen Beobachtungsinstrumentes 40 sollen die Grundprinzipien der Erfindung erläutert werden. Das Beobachtungssystem 40 weist einen Schaft 42 auf, in dem stehend ein Bildsensor 44 mit rechteckförmiger Kontur eingebaut ist.

Der Bildsensor 44 weist einen aktiven Bereich 46 auf, der asymmetrisch zu der Außenkontur des Bildsensors 44 angeordnet ist. Die Schaftachse 58 liegt exakt in der Mitte des Schaftes 42. Der Hauptstrahl 52 ist gegenüber der Schaftachse 58 in Richtung der Mittelebene 56 in der Darstellung von Fig. 5 etwas nach oben verschoben. Die erste Ebene wird durch den Hauptstrahl 52 und die Orthogonale 50 aufgespannt. Die zweite Ebene wird durch die zweite Orthogonale, die auf dem Hauptstrahl 52 steht, aufgespannt. Der aktive Bereich 46 des Bildsensors 44 ist um ein solches Maß entgegen dem Uhrzeigersinn gegenüber der Schaftachse 58 verdreht, dass die Bildfläche 60 im aktiven Bereich 46 des Bildsensors 44 liegt. Vergleicht man nun die in Fig. 2 dargestellte Lage des Bildsensors mit der in Fig. 5 dargestellten Lage, so ist zu erkennen, dass durch das Verdrehen des Bildsensors 44 die Bildfläche 60 voll im aktiven Bereich 46 liegt. Das distale Umlenkprisma (hier nicht ersichtlich) erzeugt im Linsensystem eine Bildfläche 60, die längs der Mittelebene 56 nach oben verschoben ist, jedoch symmetrisch dazu ist. Es ist möglich einen symmetrischen Sichtkegel, wie in Fig. 4 dargestellt ist, zu erzeugen und ein hochaufgelöstes Bild vollmittig im aktiven Bereich 46 des Bildsensors 44 zu erhalten, allerdings etwas verdreht und nach "oben" versetzt.

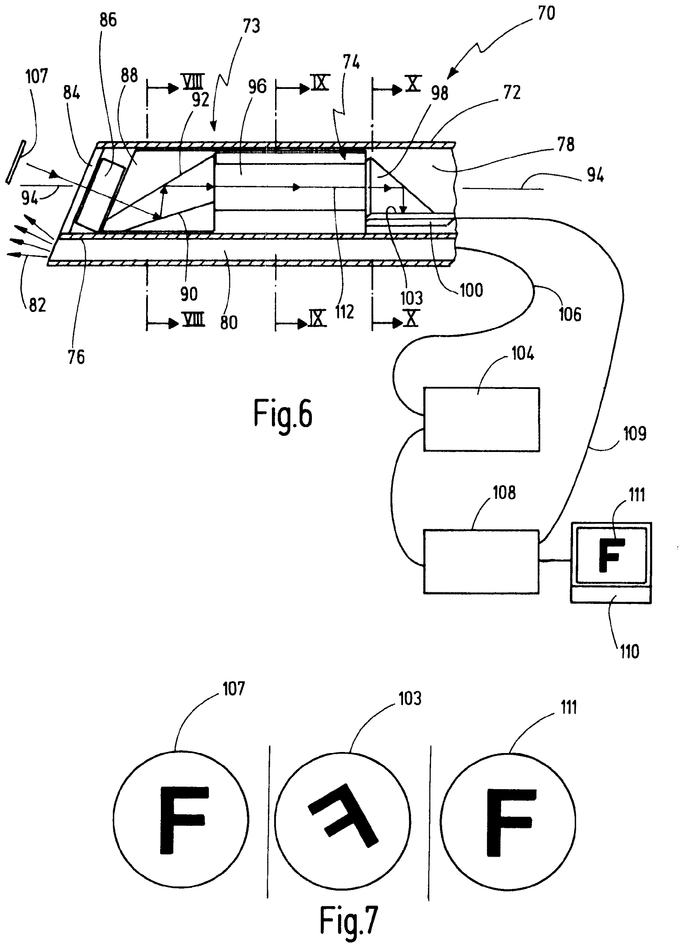

Bei einem in den Figuren 6 bis 12 dargestellten zweiten Ausführungsbeispiel ist ein Beobachtungsinstrument 70 dargestellt, das einen Schaft 72 aufweist.

Im distalen Endbereich 73 des Schaftes 72 ist ein opto-elektronisches Bildaufnehmersystem 74 aufgenommen.

Der Innenraum des Schaftes 72 ist durch einen Quersteg 76 in einen ersten Raum 78 und in einen zweiten Raum 80 aufgeteilt.

Im zweiten Raum 80 ist das Beleuchtungssystem aufgenommen meist Lichtleiter, die distalseitig aus dem zweiten Raum 80 Beleuchtungslicht 82 abgeben.

Der erste Raum 78 ist distalseitig durch ein Deckglas 84 abgeschlossen. An dieses Deckglas 84 schließt sich eine Linse 86 an, die als eine Fokussierlinse ausgebildet ist. An deren proximales Ende ist eine auf einer gegenüber der Längsachse 94 des Schaftes 72 geneigte Eintrittsfläche eines distalen Umlenkprismas 88 angebracht. Das distale Umlenkprisma 88 weist eine erste Umlenkfläche 90 und eine zweite Umlenkfläche 92 auf. Diese werden auch als Spiegelflächen bezeichnet. Dadurch wird ein Hauptstrahl 112 eines Bildes eines Objektfeldes 107 zentral mittig längs der Längsachse 94 in ein etwa stabförmiges Linsensystem 96 geführt. Am proximalen Ende des Linsensystems 96 ist ein proximales 90°-Umlenkprisma 98 angeordnet, dessen Austrittsfläche auf einem Bildsensor 100 ruht. Der Bildsensor 100 ist somit liegend eingebaut, so dass sich dessen aktiver Bereich 102 bzw. dessen Bildebene 103 parallel und etwas im Abstand zur Mittellängsachse 94 bzw. zum Hauptstrahl 112 erstreckt. Wie zuvor beschrieben ist der aktive Bereich 102 asymmetrisch zur Außenkontur des etwa rechteckförmigen Bildsensors 100 angeordnet.

In Fig. 6 ist noch zu erkennen, dass die in dem zweiten Raum 80 aufgenommenen Lichtleiter über eine Leitung 106 mit einer Lichtquelle 104 verbunden sind. Diese ist wiederum mit einer Bildverarbeitungseinheit 108 verbunden, die einerseits mit dem Bildsensor 100 und andererseits mit einem Monitor 110 verbunden ist. Die Lichtquelle 104 kann auch separat stehen.

In Fig. 6 ist der Verlauf eines Hauptstrahles 112 aufgezeichnet, der von einem Beobachtungsfeld 107 ausgeht, wie es in Fig. 7 auf der linken Seite dargestellt ist. Hier soll der große Buchstabe "F" beobachtet werden. Es ist aus Fig. 6 zu erkennen, dass die Blickrichtung etwa um 20°aus der 0°-Blickricht ung abweicht, die längs der Längsachse 94 verläuft.

Der Hauptstrahl 112 durchläuft das Deckglas 84 und die Fokussierlinse 86 und wird an der ersten Umlenkfläche 90 auf die zweite Umlenkfläche 92 des distalen Umlenkprismas 88 umgelenkt. Von der zweiten Umlenkfläche 92 wird der Hauptstrahl 112, mittig und in Richtung der Längsachse 94 verlaufend, durch das Linsensystem 96 geführt. Am proximalen Ende des Linsensystems 96 tritt der Hauptstrahl 112 in das proximale Umlenkprisma 98 ein, wird um 90°abgelenkt und trifft auf den aktiven Bereich 102 bzw. die Bildebene 103 des Bildsensors 100.

Durch die zuvor beschriebene Verdrehung entsteht ein Bild auf dem Bildsensor 100, wie es in Fig. 7 in der Mitte dargestellt ist, d.h., das "F" ist einmal seitenverkehrt gespiegelt und gedreht. In der Bildverarbeitungseinheit 108 wird das Bild dann entsprechend bearbeitet, so dass auf einem Monitor 110 ein Bild erscheint, wie es in Fig. 7 auf der rechten Seite dargestellt ist, also dem ursprünglichen Bild entspricht.

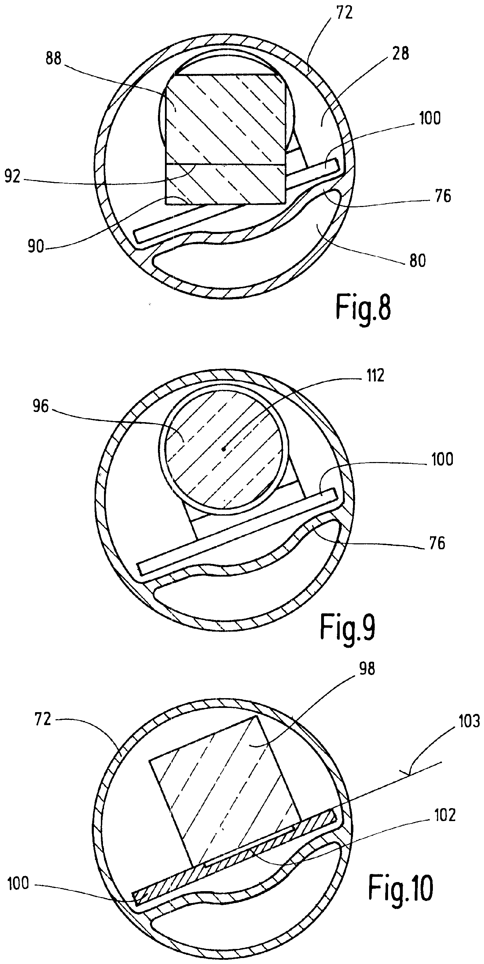

Aus den Schnittdarstellungen von Fig. 8, 9 und 10 sind die jeweiligen aus Glasmaterial dargestellten optischen Elemente ersichtlich, also das distale Umlenkprisma 88 mit seiner ersten bzw. zweiten Umlenkfläche 90 bzw. 92. In Fig. 9 ist das Linsensystem 96 mit dem Hauptstrahl 112 ersichtlich. Aus Fig. 10 ist ersichtlich, dass das proximale Umlenkprisma 98 den aktiven Bereich 102 des Bildsensors 100 bedeckt, wobei die Bildebene 103 unmittelbar unter der Auflagefläche des proximalen Umlenkprismas 98 liegt.

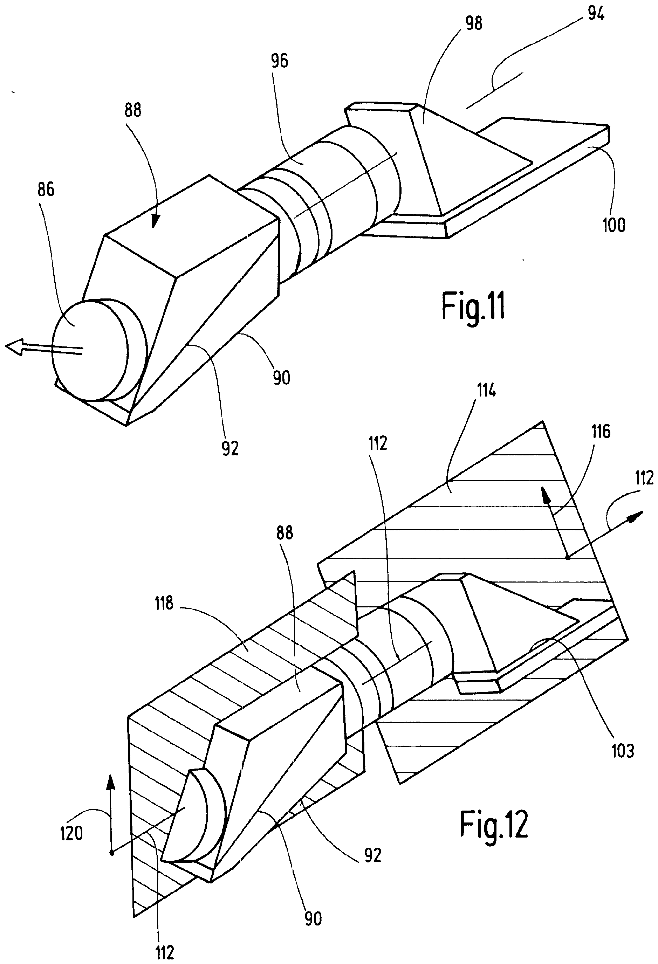

In Fig. 11 sind diese Bauelemente noch einmal zusammenhängend perspektivisch dargestellt. In Fig. 12 sind nun zusätzlich die beiden Ebenen dargestellt und wie sie zueinander verdreht sind.

Die erste Ebene 114 wird durch den Hauptstrahl 112 und einer auf der Bildebene 103 stehenden Orthogonalen 116 aufgespannt.

Die zweite Ebene 118 wird ebenfalls durch den Hauptstrahl 112 und einer zweiten Orthogonalen 120 aufgespannt, die auf den Umlenkflächen 90, 92 des distalen Umlenkprismas 88 steht.

Die erste Ebene 114 ist gegenüber der zweiten Ebene 118 nunmehr so verdreht, dass, wenn man auf den Bildsensor 100 blickt, eine Situation eintritt, wie sie in Fig. 5 dargestellt ist. Ein Unterschied zwischen der Darstellung von Fig. 12 und Fig. 5 besteht darin, dass in Fig. 12 der Bildsensor 100 liegend und nicht, wie in Fig. 5, stehend angeordnet ist. Das Grundprinzip ist aber bei beiden Ausgestaltungen gleich.

Es ist bei der zweiten Ausführung möglich die erste und die zweite Ebene 114 bzw. 118 so zueinander zu verdrehen bzw. die Umlenkflächen 90 und 92 des distalen Umlenkprismas 88 so anzuordnen, dass ein symmetrischer Sichtkegel 22 erzeugt wird, wie er in Fig. 4 dargestellt ist.

## Patentansprüche

1. Als Resektoskop gestaltetes Beobachtungsinstrument, mit einem, eine Mittelebene (12, 56) aufweisenden Hohlschaft (72) in dessen distalem Endbereich (73) ein opto-elektronisches Bildaufnehmersystem (74) angeordnet ist, das bildeintrittsseitig ein distales Umlenkprisma (88) aufweist, dessen Umlenkflächen (90, 92) ein Bild in ein optisches Linsensystem (96) leiten, das das Bild auf einen proximal vom Linsensystem (96) angeordneten asymmetrischen Bildsensor (100) auf dessen Bildebene (103) abbildet, und mit zwei seitlichen Stangen (26, 28), die über eine Resektoskopschlinge (30) miteinander verbunden sind, wobei der Bildsensor (100) einen asymmetrischen aktiven Bereich (102) aufweist, der asymmetrisch zur Außenkontur des Bildsensors (100) liegt, wobei der Bildsensor (100) gekippt gegenüber der Schaftachse (58) angeordnet ist, wobei zwischen dem proximalen Ende des Linsensystems (96) und dem Bildsensor (100) ein proximales Umlenkprisma (98) angeordnet ist, das das vom optischen Linsensystem (96) austretende Bild auf den gekippten Bildsensor (100) umlenkt, und wobei der Hauptstrahl (112) zentral mittig längs einer Längsachse (94) des Linsensystems (96) geführt wird, wobei eine erste Ebene (114) durch den Hauptstrahl (112) und eine erste Orthogonale (116) auf der Bildebene (103) des Bildsensors (100) aufgespannt ist, und eine zweite Ebene (118) durch den Hauptstrahl (112) und eine zweite Orthogonale auf den Umlenkflächen (90, 92) des distalen Umlenkprismas (88) aufgespannt ist, wobei die erste Ebene (114) und die zweite Ebene (118) derart um den Hauptstrahl (112) zueinander verdreht sind, dass ein zu der Mittelebene (12) des Hohlschafts (72) symmetrischer Sichtkegel (22) erzeugbar ist, dessen Bildfläche (60) voll im aktiven Bereich (102) des Bildsensors (100) liegt, so dass Sichtstellen (27', 29') des Sichtkegels (22) mit den Stangen (26, 28) auf derselben axialen Höhe liegen, und wobei der Hauptstrahl (112) gegenüber einer Schaftachse (58) des Hohlschaftes (72) verschoben ist.

2. Beobachtungsinstrument nach Anspruch 1, **dadurch gekennzeichnet, dass** der Bildsensor (100) liegend und parallel zur Längsachse (94) angeordnet ist, und dass das proximale Umlenkprisma (98) eine 90°-Umlenkung des Bildes bewirkt.

3. Beobachtungsinstrument nach einem der Ansprüche 1 oder 2, **dadurch gekennzeichnet, dass** die erste und die zweite Ebene (114, 118) derart zueinander verdreht sind, dass der Sichtkegel (22) symmetrisch zur Schaftachse (58) verläuft.

4. Beobachtungsinstrument nach einem der Ansprüche 1 bis 3, **dadurch gekennzeichnet, dass** der Bildsensor (100) mit einer Bildverarbeitungseinheit (108) verbunden ist, die das durch die Verdrehung verdrehte und ggf. verschobene Bild wieder seiten- und höhenrichtig aufrichtet.

5. Beobachtungsinstrument nach einem der Ansprüche 1 bis 4, **dadurch gekennzeichnet, dass** das opto-elektronische Bildaufnehmersystem (74) derart aufgebaut ist, dass eine von der 0°-Blickrichtung abweichende Blickrichtung erfolgt.

## Claims

1. An observation instrument that is arranged as a resectoscope, the instrument comprising a hollow shaft (72), which has a center plane (12, 56) and in the distal end region (73) of which there is arranged an opto-electronic image pick-up system (74), which comprises a distal deflection prism (88) on the image entrance side, whose deflection surfaces (90, 92) guide an image into an optical lens system (96), which images the image onto an asymmetrical image sensor (100) that is arranged proximally of the lens system (96) on the image plane (103) thereof, and comprising two lateral rods (26, 28) which are connected to one another via a resectoscope loop (30), wherein the image sensor (100) comprises an asymmetrical active region (102), which lies asymmetrically with respect to the outer contour of the image sensor (100), wherein the image sensor (100) is arranged tilted with respect to the shaft axis (58), wherein a proximal deflection prism (98) is arranged between the proximal end of the lens system (96) and the image sensor (100), which deflects the image emerging from the optical lens system (96) onto the tilted image sensor (100), and wherein the main beam (112) is guided centrally centered along a longitudinal axis (94) of the lens system (96), wherein a first plane (114) is spanned by the main beam (112) and a first orthogonal (116) on the image plane (103) of the image sensor (100), and a second plane (118) is spanned by the main beam (112) and a second orthogonal on the deflection surfaces (90, 92) of the distal deflection prism (88), wherein the first plane (114) and the second plane (118) are rotated relative to one another about the main beam (112) in such a way that a viewing cone (22) can be generated, which is symmetrical to the center plane (12) of the hollow shaft (72), and whose image area (60) lies fully in the active region (102) of the image sensor (100), so that viewing points (27', 29') of the viewing cone (22) are at the same axial height as the rods (26, 28), and wherein the main beam (112) is displaced with respect to a shaft axis (58) of the hollow shaft (72).

2. The observation instrument according to claim 1, **characterized in that** the image sensor (100) is arranged in a lying orientation and parallel to the longitudinal axis (94), and **in that** the proximal deflection prism (98) causes a 90° deflection of the image.

3. The observation instrument according to any one of the claims 1 or 2, **characterized in that** the first and second planes (114, 118) are rotated relative to one another in such a way that the viewing cone (22) extends symmetrically with respect to the shaft axis (58).

4. The observation instrument according to any one of the claims 1 to 3, **characterized in that** the image sensor (100) is connected to an image processing unit (108), which erects the image, which has been rotated and possibly shifted by the rotation, laterally and vertically correct again.

5. The observation instrument according to any one of the claims 1 to 4, **characterized in that** the opto-electronic image pick-up system (74) is configured in such a way that the direction of view deviates from the 0° direction of view.

## Revendications

1. Instrument d'observation conçu comme un résectoscope, comportant un arbre creux (72) qui présente un plan médian (12, 56) et dans la zone d'extrémité distale (73) duquel est disposé un système optoélectronique d'acquisition d'image (74) qui comporte un prisme de déviation distal (88) du côté de l'entrée d'image, dont les surfaces de déviation (90, 92) acheminent une image dans un système de lentilles optique (96) qui forme l'image sur un capteur d'image asymétrique (100) disposé à proximité du système de lentilles (96) sur le plan image (103) de celui-ci, et comportant deux tiges latérales (26, 28) qui sont reliées entre elles par une bride de résectoscope (30), dans lequel le capteur d'image (100) présente une zone active asymétrique (102) qui est asymétrique par rapport au contour extérieur du capteur d'image (100), dans lequel le capteur d'image (100) est disposé de manière inclinée par rapport à l'axe d'arbre (58), dans lequel il est prévu entre l'extrémité proximale du système de lentilles (96) et le capteur d'image (100) un prisme de déviation proximal (98) qui dévie l'image sortant du système de lentilles optique (96) vers le capteur d'image (100) incliné, et dans lequel le faisceau principal (112) est dirigé de manière centrale le long d'un axe longitudinal (94) du système de lentilles (96), dans lequel un premier plan (114) est défini par le faisceau principal (112) et une première perpendiculaire (116) au plan image (103) du capteur d'image (100), et un second plan (118) est défini par le faisceau principal (112) et une seconde perpendiculaire aux surfaces de déviation (90, 92) du prisme de déviation distal (88), dans lequel le premier plan (114) et le second plan (118) sont tournés l'un par rapport à l'autre autour du faisceau principal (112) de telle sorte qu'un cône d'observation (22) symétrique de l'arbre creux (72) par rapport au plan médian (12) puisse être généré, cône dont la surface d'image (60) se trouve entièrement dans la zone active (102) du capteur d'image (100), de sorte que des points d'observation (27', 29') du cône d'observation (22) se trouvent à la même hauteur axiale que les tiges (26, 28), et dans lequel le faisceau principal (112) est déplacé par rapport à un axe d'arbre (58) de l'arbre creux (72).

2. Instrument d'observation selon la revendication 1, **caractérisé en ce que** le capteur d'image (100) est disposé horizontalement et parallèlement à l'axe longitudinal (94), et **en ce que** le prisme de déviation proximal (98) provoque une déviation de l'image de 90°.

3. Instrument d'observation selon l'une des revendications 1 ou 2, **caractérisé en ce que** les premier et deuxième plans (114, 118) sont tournés l'un par rapport à l'autre de telle sorte que le cône d'observation (22) soit symétrique par rapport à l'axe d'arbre (58).

4. Instrument d'observation selon l'une des revendications 1 à 3, **caractérisé en ce que** le capteur d'image (100) est relié à une unité de traitement d'image (108) qui redresse l'image ayant été tournée et éventuellement décalée par la rotation, en respectant le sens et la hauteur.

5. Instrument d'observation selon l'une des revendications 1 à 4, **caractérisé en ce que** le système optoélectronique d'acquisition d'image (74) est réalisé de manière à obtenir une direction de visée s'écartant de la direction de visée 0°.
